# EUROPEAN PATENT SPECIFICATION

(11) **EP 1 251 879 B1**
(45) Date of publication and mention of the grant of the patent: **30.06.2004**
(21) Application number: 01905513.6
(22) Date of filing: 01.02.2001
(51) Int. Cl.: A61L 2/07, A01G 1/04

(54) **METHOD AND APPARATUS FOR STERILISING AND/OR PASTEURISING GROWTH MEDIA**
VERFAHREN ZUR STERILISIERUNG UND/ODER PASTEURISIERUNG VON WACHSTUMSMEDIEN UND VORRICHTUNG FÜR DIESES VERFAHREN
PROCEDE ET APPAREIL UTILISES POUR STERILISER ET/OU PASTEURISER DES MILIEUX DE CROISSANCE

(30) Priority: 01.02.2000 FR 0001334
(43) Date of publication of application: 30.10.2002
(73) Proprietor: Vandenhove, Frans, 3840 Rijkel-Borgloon (BE)
(72) Inventor: Vandenhove, Frans, 3840 Rijkel-Borgloon (BE)
(74) Representative: Bird, Ariane
(86) International application number: PCT/BE2001/000016
(87) International publication number: WO 2001/056617

(56) References cited:
- EP-A- 0 931 553
- WO-A-98/48853
- GB-A- 2 002 645
- US-A- 5 406 747
- PATENT ABSTRACTS OF JAPAN vol. 016, no. 228 (C-0944), 27 May 1992 (1992-05-27) & JP 04 045719 A (TAKUMI KAGAWA), 14 February 1992 (1992-02-14) cited in the application

## Description

The present invention relates to a method of at least partly sterilising or pasteurising material, especially organic material such as growth media and nutrient layers, in particular for plants and mushrooms, but also other types of wastes such as hospital wastes; and to equipment for implementing the method.

### Technical Background

Mushrooms (cultivated or wild) and some plants, in particular vegetables (for example endive, tomato), fruit (for example, raspberry, kiwi fruit) and fruit trees, are habitually grown by placing them on a substrate such as a growth medium or a nutrient layer to encourage their growth or, in the case of plant seeds to make them germinate. A great variety of organic materials can be used for the substrate, for instance, peat, soil, sawdust, straw, hay, plant waste (corn, wood), wood chips (for example hedge clippings), cereals (for example, wheat, rye, millet) and the like. White mushrooms are also habitually grown on a top layer of a nutrient medium such as peat. It is usually necessary to at least partly sterilise or completely pasteurise the substrate before it is used in order to eliminate harmful bacteria, germs, microbes, micro-organisms, worms and other undesirable infectious agents that could contaminate the mushrooms and/or the plants grown on the medium. It is also necessary to cool said substrate after sterilising or pasteurising it so that it can be packaged in the sterile state or, in the case of mushrooms, seeded.

Various methods and apparatus have been described for sterilising or pasteurising organic substances. Generally, it is desired to pasteurise organic material while maintaining or providing a certain moisture content. A simple device is described in US 4,915,606 which involves placing the growth medium in a steam oven. This approach requires a considerable time before the sterilisation process has penetrated into the core of any large pieces of the medium. It would better to break up the pieces of the growth medium and to stir it continuously. This has been attempted partly in an apparatus described in BE 890 468. It makes use of a simple rotating drum for tumbling the material. To provide more active stirring, addition of an internal screw is proposed in GB 2 002 645, DE 43 15 660 and DE 699 387. A further refinement is described in EP 276645 in which a centrally located screw runs in a trough and the material can be discharged from the outer hollow drum by reversing the direction of the screw.

None of the above machines provides satisfactory reproducibility of the uniformity of the sterilised or pasteurised growth medium. There is no guarantee that small volumes of the processed material are still not properly pasteurised.

An alternative approach is to try and process materials within a long tube having an internal screw as disclosed in EP 931 553, US 5,406,747 or JP 4045719. Such apparatus may guarantee that the material follows a well-defined path and is continuously stirred by the drive screw. A disadvantage of this approach is that a very long tube (or tubes) is (are) required or a very low throughput is obtained if the appreciable residence times for sterilisation or pasteurisation times are to be obtained. Such a long tube is not suitable for the cramped production space often encountered in small and medium size enterprises. As a partial solution to these problems WO 98/48853 proposes using two tubes at an angle to each other and a holding chamber placed at the junction of the two for allowing a dwell time of the material. The total machine still takes up a great deal of space for a very little chamber volume and therefore a low throughput. Due to the large exposed heated surface area, heat loss is large resulting in low heat efficiency.

The present invention aims to solve the above problems in a simple and reliable manner and without recourse to a device that is costly or difficult to maintain despite the fact that the confusing variety of unsuccessful known solutions provides no clear indication of a preferred development direction.

Hence, a first object of the present invention is to provide a simple, reliable (reproducible) and effective (high yield in minimum time) method of obtaining a substrate such as a growth medium for plants and mushrooms that is at least partly sterilised or completely pasteurised. The sterilised or pasteurised substrate is then cooled for sterile packaging or seeding.

A second object of the present invention is to provide equipment for obtaining an at least partially sterilised or pasteurised substrate such as a growth medium for plants and mushrooms that is economical to construct, maintain and use. To be more specific, one object of the invention is to provide an apparatus whose design concentrates on simplifying the hardware and reducing the power required to operate it.

### Summary of the Invention

The present inventor has determined that adequate pasteurisation cannot be achieved if the treatment time/process of the growth medium varies significantly from one portion to another within the medium in the same load. The present inventor has determined for the first time why commercially available rotary machines suffer from this problem and has developed a solution which has been extensively tested. The design of this commercial equipment is such that some parts of the material to be processed experience a different level of pasteurisation or sterilisation, e.g. less pasteurisation time, than others. The consequence is incomplete pasteurisation of these parts and poor quality (non-homogeneity) of the corresponding processed material.

In a first aspect, the present invention provides a discontinuous - or batch - method of homogeneously and at least partially sterilising or homogeneously and completely pasteurising a substrate as defined in appended claim 1; in said method:
- in a first step, said substrate is loaded into a hollow vessel, the hollow vessel being provided with means for at least partly sterilising or pasteurising the substrate;
- in a second step the hollow vessel is rotated and heat energy is supplied to the substrate, during the rotation of the vessel the substrate is transported towards an end of the vessel in a first direction through a tubular section located within the hollow vessel, the tubular section having a first and a second end;
- in a third step, the substrate is discharged from the second end of the tubular section into the rotating hollow vessel and is transported in a second direction opposite to that of the first direction in the hollow vessel until it reaches the first end of the tubular section, the substrate transported in the first direction being separated from the substrate transported in the second direction by a wall of the tubular section, and
- the second and third steps are repeated to obtain homogeneous and at least partial sterilisation or homogeneous and complete pasteurisation of said substrate.
Preferably the substrate is introduced at an end of the hollow adjacent the first end of the tubular section. A fluid for moistening said substrate may be optionally introduced into or with the substrate as it is introduced into the vessel. The hollow vessel may be sealed during processing which allows pressurisation of the vessel. To remove the processed substrate, preferably following an internal or external, cooling step, it is discharged from the machine for subsequent packaging in the sterile state or, if the substrate is a mushroom growth medium, for seeding in a sterile chamber. In an advantageous variant of the method according to the invention, sterilised or pasteurised mushroom growth medium can be seeded inside the apparatus, after it has cooled, before the step of discharging it from said machine.

In a first preferred embodiment of the method according to the invention, transport in the first and second directions is concentric and, more particularly, coaxial with the rotation axis of the vessel. In another preferred embodiment of the method the first end of tubular section has an opening for allowing the substrate access to the transport device within the tubular section and hence transfer into the tubular section. This opening may be provided by the upper section of a trough which partly surrounds the transport device. The trough and the tubular section are preferably co-axial. The length of the opening above the trough compared to the length of the tubular section is such that homogeneous pasteurising is obtained. The length of this opening defines the ease of access to the tubular section and can therefore determine the residence times in the tubular section and/or in the hollow vessel.

Depending on the desired pasteurisation temperature and optionally on the altitude at which the method is carried out, the second and third steps of the method according to the invention are preferably carried out at a pressure slightly greater than atmospheric pressure. A simple way to achieve this is to introduce a moistening fluid into the hollow vessel in a sealed state before heating - the vapour generated by heating the liquid will then pressurise the vessel. To save time however; it is preferred to introduce moistening fluid at a high temperature - this reduces the time to heat up at the start, and hence reduces overall process time. For example, when water is used boiling water may be introduced into the vessel at the start.

In a preferred embodiment of the method according to the invention the tubular section is preferably coaxial with the rotation axis of the vessel and the tubular section includes within it a transport device, e.g. a transport screw. In the context of the present invention, the expression "tubular section" means any conduit, regardless of the shape of its cross-section (triangular, square, rectangular, pentagonal, hexagonal, circular, oval, elliptical, polygonal or any other shape). If required, the wall of the tubular section can be perforated provided that the number and/or the size of the holes does not interfere with the prime function of the section, which is separation of the material flows in the first and second direction, e.g. the holes should not allow substantial mixing of the two flows. The tubular partition is preferably provided with a cross-section which avoids clogging in the tubular section. For example, the tubular section may have a cross-section with sharp edges or ridges on the inside of the wall to prevent sticking (non-circulation) of the substrate in the tubular section.

The transport device in the tubular section is preferably one whose direction can be reversed, such as a transport screw. The rotation speeds of the hollow vessel, on the one hand, and the transport device in the tubular section, on the other hand, can be the same or different but are preferably settable. Also, the transport speed of the transport device inside the tubular section can be varied during processing, and in particular transport can be stopped as soon as the machine operator is satisfied with the homogeneity obtained. However, the rotation speed (expressed in revolutions per minute or rpm) of the vessel is most often adjusted to be less than that of the transport device. By way of example only, the rotation speed of the vessel may be in the range 0.5 rpm to approximately 5 rpm, and that of the transport device may be up to approximately 100 rpm, depending on various factors such as its diameter. The means for at least partly sterilising or pasteurising the substrate are preferably housed in a part of the apparatus such that at least partial sterilisation or pasteurisation begins at least during the third step of the method according to the invention, and preferably as early as the second step. For example, this is conveniently achieved by providing equipment for maintaining a sterilising or pasteurising temperature on the inside wall of the hollow vessel and/or on or in the tubular section itself, as described in more detail hereinafter. However, other equipment may be provided, e.g. steam injection.

The geometrical dimensions, in particular the longitudinal and transverse dimensions, such as the length and the diameter - in the case of a hollow vessel - or the width, of the various components of the apparatus, and the rotation speeds of the vessel and the transport device within it, are preferably chosen to operate in conjunction so that the time for which each fraction of the substrate is effectively subjected to the sterilising or pasteurising conditions is uniform in each part of the machine throughout the processing, so contributing to a homogeneous quality of the end product.

In the context of the present invention, the expression "moistening fluid" means a gaseous or preferably liquid fluid which can be mixed with the substrate and, by being mixed homogeneously with the substrate, can constitute a medium that can be completely pasteurised or at least partly sterilised. For example the fluid may be water at a temperature from approximately 5°C to approximately 95°C, or steam. The quantity of moistening fluid employed is determined by the nature of the substrate (which varies considerably from one growth medium to another), by the moisture content of the substrate before it is loaded into the sealed rotary machine, and by the texture required for easy transportation of the substrate moistened in said machine. By way of example, the quantity of moistening fluid introduced is preferably such that the proportion of dry material in the sealed rotary machine is from approximately 20% by weight to approximately 60% by weight, i.e. so that the water/substrate weight ratio is from approximately 2:3 to approximately 4:1. Depending on the nature of the substrate to be treated by the method according to the invention, it is not always necessary to employ completely sterilising conditions.

In accordance with a second aspect of the present invention, an apparatus for at least partly sterilising or pasteurising a substrate such as a growth medium for plants or mushrooms is provided and is defined in appended claim 12. It comprises:
- a hollow vessel provided with means for its rotation about a rotation axis, and with means for at least partly sterilising or completely pasteurising the substrate;
- an opening for loading the substrate into the vessel;
- a first transport device for transporting the substrate in a first direction and extending throughout a substantial portion of the interior of the hollow vessel,
- a tubular partition surrounding the first transport device and having a wall forming a separator between a zone comprised inside the tubular partition and a zone comprised between said tubular partition and the inner wall of the hollow vessel,
- a means for discharging the at least partly sterilised or completely pasteurised substrate from the tubular partition into a hollow space of the hollow vessel, and
- a second transport device for directing the substrate discharged from the tubular partition to follow a path in a second direction opposite to the first direction.
Further, the vessel may be provided with means for introducing a moistening fluid into the vessel. The vessel preferably has a means for discharging the substrate after processing. The vessel may also be sealed during processing, for example by a valve disposed at one end of the vessel (preferably the same end as loading is carried out) to load the substrate and/or discharge the pasteurised or sterilised substrate.

For reasons of efficiency, the tubular partition and the hollow vessel are elongate, and for example cylindrical or quasi-cylindrical, parallelepiped or quasi-parallelepiped, and the method of the invention is preferably carried out with the rotation axis of the vessel being substantially horizontal or with a tilt of less than 25° and, for example, 5 or 10° downwards towards the first end of the tubular section.

As previously indicated with regard to the method forming the first aspect of the present invention, the separator partition of the above equipment is a tubular partition, such as a tubular passage having a longitudinal axis parallel or coaxial with the rotation axis of the vessel, and separating the transport zone for transporting the substrate inside the tubular passage, on the one hand, and the transport of the substrate in the transport zone outside the tubular passage in the reverse direction, between it and the inside wall of the vessel, on the other hand.

The means for introducing a moistening fluid preferably take the form of a simple inlet pipe for said fluid inside the vessel, for example a tube at the end of the vessel opposite the means for loading and discharging the substrate. The means for at least partly sterilising or completely pasteurising the substrate can take the form, for example, of at least one jacket for circulating a hot fluid, such as steam or water maintained at a temperature from, for example, approximately 60°C to approximately 140°C, disposed on the wall of the vessel. The same means could be used, after sterilisation or pasteurisation of the substrate has been completed, to cool the sterilised or pasteurised substrate by circulating a cold fluid, such as water at a temperature from approximately 5°C to approximately 30°C (depending on local climatic conditions), for the time necessary to cool the substrate to a temperature compatible with sterile packaging or, in the case of a substrate constituting a mushroom growth medium, for seeding the substrate.

In a preferred embodiment of the invention, the transport device passes through a hole in the end of the vessel. By reversing the transport device, the processed substrate may be discharged from the vessel through this hole. In another variant of the present invention, the tubular section defined by the tubular partition can be provided with means for sterilising or pasteurising the moistened substrate, for example a jacket surrounding said tubular section over a substantial part of its length for circulating a hot fluid as previously defined, in order to increase the surface area of contact and therefore the transfer of heat between the substrate to be sterilised or pasteurised and said hot fluid and consequently to increase the efficiency of the operation. The hot fluid circulation jacket around the tubular section can, if necessary, communicate with the jacket on the wall of the vessel to sterilise or pasteurise the moistened substrate, or can even replace it.

In accordance with the present invention, the transport device can be a transport screw or a spiral conveyor. In accordance with the present invention, the transport device is preferably provided with a reversible drive means such as a motor, for example a reversible electric or hydraulic motor.

The invention will now be described with reference to the accompanying figures which are provided by way of example only and with no intention of limiting the present invention.

### Brief Description of the drawings

Figure 1 is a partly cutaway diagrammatic side elevation view of an apparatus according to an embodiment of the present invention.
Figures 2a, b, c,d show cross-sections through parts of the vessel such as trough and tubular sections of the embodiment of Figure 1.

### Description of the illustrative embodiments

The present invention will be described with reference to certain embodiments and drawings but is not limited thereto but only by the claims. The present invention will mainly be described with reference to pasteurising growth media for mushrooms but the present invention is not limited thereto and may include sterilising wastes such as hospital waste.

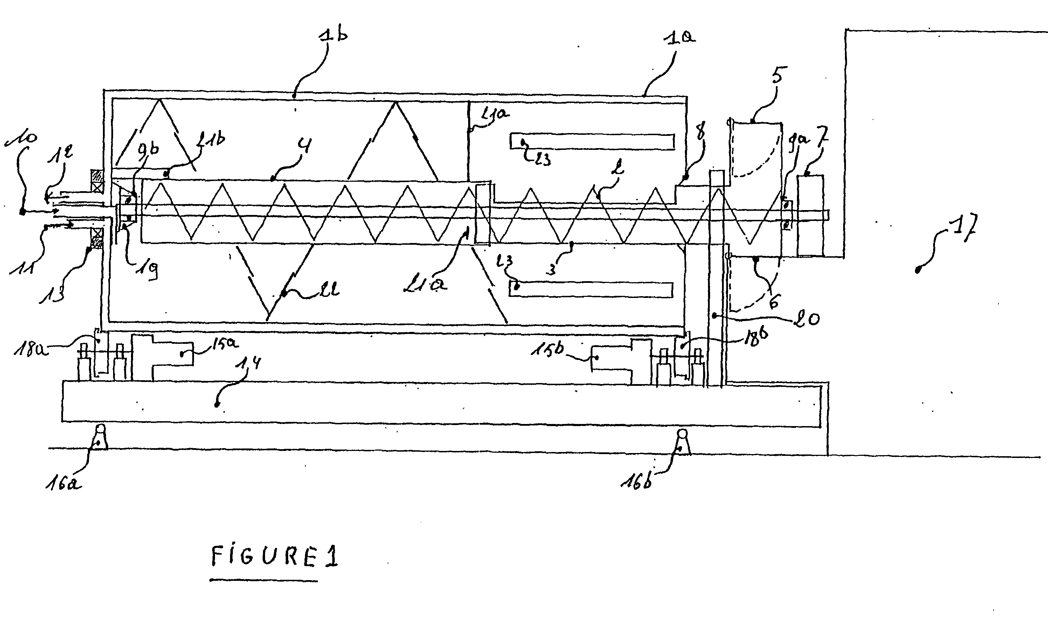

An apparatus for partial sterilisation or pasteurisation in accordance with an embodiment of the present invention will be described with reference to Figure 1. The apparatus includes a hollow, e.g. cylindrical vessel (1a) which can be rotated about a substantially horizontal or inclined rotation axis. It may be rotated by two pairs of drive wheels (15a) and (15b) fixed to respective supports (18a) and (18b). The vessel (1a) has at one end a system preferably including a sealed valve (5) for loading the material to be sterilised or pasteurised (from a storage and/or transport system, for example a hopper, not shown in Figure 1). The seal between the loading system and the vessel (1a) may be provided by a rotary seal (8). A transport device such as an Archimedes screw or spiral transporter (2) below the valve.(5) and constrained to rotate with the vessel (1a) by fixing means, in this instance a cone (19) and a sealed bearing (9b) fixed to said cone, extends almost the entire length of the vessel (1a) and the entire length of the aforementioned loading system. This transport device is provided for controlled and regular transport of the material. The sealed bearing (9b) holds the Archimedes screw (2) in position and allows for any differential expansion between the components. The end of the Archimedes screw (2) at the same end as the loading system is driven by a reversible hydraulic or electric motor (7). The seal between the loading system and the shaft driven by the motor (7) is provided by a rotary seal (9a) with a bearing. A cylindrical trough (3) coaxial with the Archimedes screw (2) and of slightly larger radius than the Archimedes screw (2) receives the material to be sterilised or pasteurised from the loading system. The trough (3) is fastened to the frame (14) by fixing means (20). A tubular passage (4) fixed to the vessel (1a) by fixing means (21a) and (21b) is coaxial with the Archimedes screw (2) and its radius is at least equal to that of the trough (3). The tubular passage (4) extends a substantial portion of the length of the vessel (1a) and may overlap slightly beyond the end of the trough (3). The trough has an open top, the length of this opening being preferably less than the length of the tubular passage.

Furthermore, the wall of the vessel (1a) may be provided with a jacket (1b) for circulating water or steam or other heating fluid such as hot oil. The jacket is preferably made from a corrosion-resistant material depending on the composition, temperature and quality of the circulating fluid. The jacket (1b) has an inlet (11) for example, connected to a water supply and a outlet (12) at the same or another end of the vessel (1a). These are conveniently placed opposite the end for loading the material to be sterilised or pasteurised. The seal between the fluid circulation/injection system (10,11,12) and the vessel (1a) is provided by a rotary seal (13) made of a material capable of resisting the temperature of the circulating fluid for a long time. Suitable materials include natural rubber, butyl rubber, polytetrafluoroethylene (for example Teflon®), bronze or a ceramic. The apparatus according to the invention further includes a tube (10) for admitting fluid into the interior of the vessel (1a), said tube preferably being located at the end of the vesssel (1a) opposite the end for loading the material to be sterilised or pasteurised, i.e. near the inlet (11) and the outlet (12) for circulating heating fluid. Finally, the Figure 1 shows two pairs of load cells (16a) and (16b) for weighing the water and the growth medium to be treated in the vessel. Although the present invention has been described with reference to a heating jacket 1b, other heating may be used such as steam injection, infra red radiators, or direct electrical heating using heating elements attached to the vessel wall..

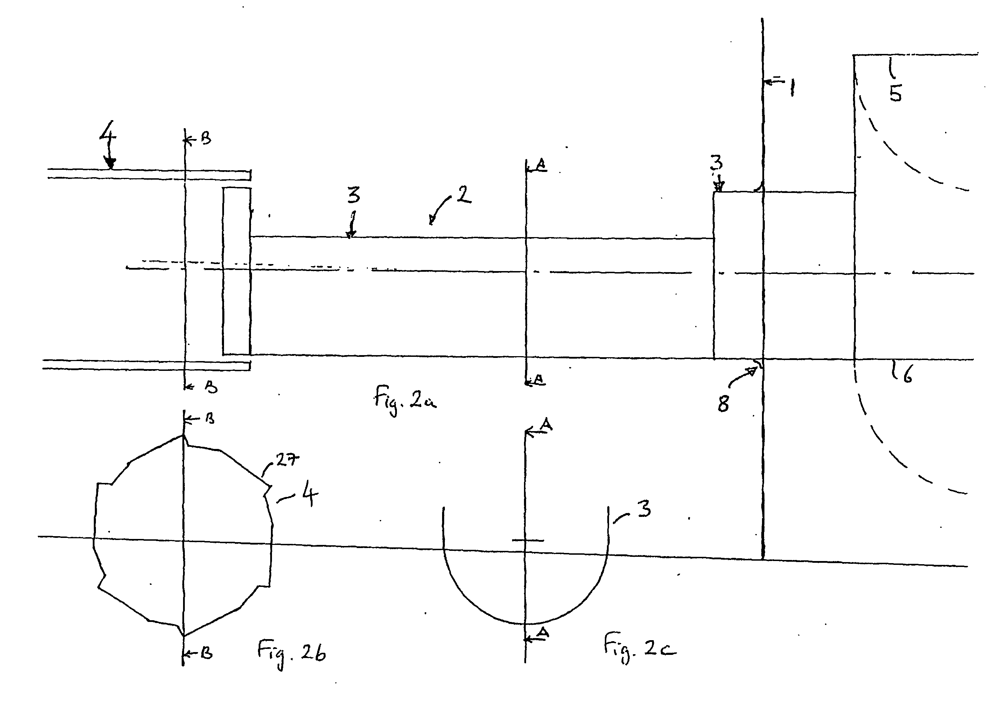

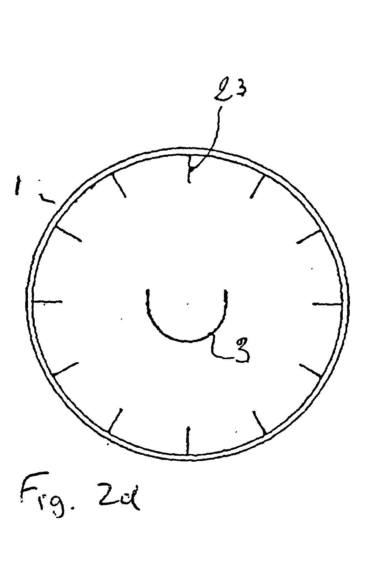

The Archimedes screw (2) rotates inside the tubular passage (4) - itself rotating at the same time as the vessel (1a) whereas the trough 3 remains stationary. The tubular passage (4) is defined by a wall constituting a separator between the zone of transport towards the front (inside the passage) and the zone of transport towards the rear (outside the passage, between it and the inner wall of the water circulation jacket (1b)). The wall defining the tubular section may be solid or perforated. However, if perforated the number and/or the size of the holes preferably should not interfere with the prime function of separation of the material flows towards the front and back of the machine, e.g. the holes should not allow substantial mixing of the two flows. The holes should preferably be small enough that material does not exit from the tubular passage into the vessel until it has reached the end of the tubular passage (4). Detailed cross-sections through the tubular section and the trough section are shown in Figures 2a, b, c. As shown in Fig. 2b, the cross-section of the tubular section may be provided with ridges or other shapes to prevent clogging of the screw (2) in the tubular section.

A second sealed valve (6) near the sealed valve (5) of the loading system and below the Archimedes screw (2) is used to discharge the sterilised or pasteurised material from the vessel, after it has cooled to ambient temperature, by reversing the direction of the Archimedes screw. The material may be discharged into a sterile enclosure (17) from which a transport system (not shown in Figure 1) may convey it to a packaging device such as a packaging machine. The rotary seal (8), the valves (5,6) and the trough (3) may be an integral piece of equipment. In variants of this embodiment that is not shown in Figure 1, the hot fluid inlet tube (10) may located in other positions, e.g. between the sealed valves (5) and (6).

The apparatus operates in the following manner: in a first step a moisturising fluid, e.g. hot water, the quantity and temperature of which have been determined beforehand, depending on the nature and the moisture content of the material to be processed, is introduced into the vessel (1a) by means of the pipe (10) and the increase in weight determined by means of the pairs of load cells (16a) and (16b). During this time hot water, or where applicable steam, hot oil or other hot fluid, is introduced via the water inlet (11) and circulated in the jacket (1b) to the fluid outlet (12). In a second step, the solid material to be at least partly sterilised or pasteurised, for example a growth medium for plants or mushrooms, is loaded into the vessel (1a) via the sealed valve (5) by means of a transport system (not shown in Figure 1). At the same time, the motor (7) is started to drive the Archimedes screw (2) in such a rotational direction that the material is transported from the loading area into the vessel and towards the remote end thereof. The rotation of the screw entrains and transports the material that has just been loaded, towards and through the trough (3) and then to and through the tubular passage (4). At the remote end of the tubular passage 4, near the inlet tube (10), the material is discharged into the hollow space of the vessel. The material is then forced to follow an opposite path, i.e. to be transported towards the rear, in a substantial portion of the annular volume between the tubular passage (4) and the wall of the jacket (1b), by virtue of the rotation of the latter. A further transport device e.g. spiral inclined blades (22), is provided on the inside wall of the hollow vessel to assist in this backwards movement. The movement in the reverse direction is preferably carried out in a controlled and regular way. Further, this second transport device should preferably allow tumbling of the material. When the material reaches the first end of the vessel (1a) it enters the trough (3) through its opening at the top. This entry may be assisted by assisting means provided by, for example, blades (23) shown scematically in Figure 2d in cross-section. In the annular volume between the tubular passage and the inner wall of the vessel, which may be referred to as an "zone of transport towards the rear", the temperature of the moistened solid material is changed to or maintained at a value suitable for it to be sterilised or pasteurised by the hot water, steam, oil or other hot fluid circulating in the jacket (1b). At the end of this transport and because of the rotation of the vessel (1a), the returning material being sterilised or pasteurised drops into the trough (3), where the Archimedes screw (2) transports it again towards the front of the vessel (1a) thus providing recycling of the material. In this way the solid material is constantly and completely mixed in the vessel (1a), in particular by dropping into the trough (3) and being conveyed towards the front by the screw (2) inside the tubular passage (4), whilst undergoing the sterilisation or pasteurisation resulting from the chosen temperature and duration of the operation. In accordance with an aspect of the present invention the material is transported in a tubular passage in a first step and then returned in a tumbling manner to the start of the tubular passage whereby the material may not tumble into the tubular section so that material cannot take a "short cut". This combination provides uniform sterilisation and pasteurisation because all material is forced to take the same path. However, the vessel is of small size, making efficient use of all volume, and the heated surface area is kept to a minimum.

Depending on the nature of the solid material treated, when it is considered that sterilisation or pasteurisation is sufficiently complete, the hot fluid circulating in the jacket (1b) is replaced with cold fluid, the vessel (1a) continuing to rotate if necessary. This cools the sterilised or pasteurised material before it is discharged or, in the case of mushroom growth medium, before sterile seeding thereof with the usual quantity of mushroom seed - from approximately 0.5% to approximately 5% by weight of the growth medium. One aspect of the present invention is a cooling step carried out in the sealed vessel 1, that is a sterile cooling step.

The motor (7) then reverses the direction of rotation of the Archimedes screw (2) in order to transport the sterilised or pasteurised and cooled material from the trough (3) to the sterile enclosure (17) via the valve (6). By rotating the vessel and operating the screw (2) in the reverse direction all the material is discharged from the vessel.

As an alternative to the embodiments that have just been explained, it is also possible, without departing from the defined scope of the present invention, to moisten the substrate to be sterilised or pasteurised before it is introduced into the vessel (1a) by means of the sealed valve (5) or between the sealed valves (5) and (6), in which case the fluid inlet pipe (10) is of no utility and can be omitted.

While the invention has been shown and described with reference to preferred embodiments, it will be understood by those skilled in the art that various changes or modifications in form and detail may be made without departing from the scope and spirit of this invention. For example, the vessel may be pressurised during processing, e.g. by the introduction of pressurised air or by sealing the vessel during heating which will automatically raise the pressure.

## Claims

1. A discontinuous - or batch - method of at least partially sterilising or homogeneously and completely pasteurising a substrate, in which method:
- in a first step, said substrate is loaded into a hollow vessel, the hollow vessel being provided with means for at least partly sterilising or pasteurising the substrate;
- in a second step the hollow vessel is rotated and heat energy is supplied to the substrate, during the rotation of the hollow vessel the substrate being transported towards an end of the hollow vessel in a first direction through a tubular section located within the hollow vessel, the tubular section having a first and a second end;
- in a third step, the substrate is discharged from the second end of the tubular section into the rotating hollow vessel and is transported in a second direction opposite to that of the first direction in the hollow vessel until it reaches the first end of the tubular section, the substrate transported in the first direction being separated from the substrate transported in the second direction by a wall of the tubular section; and
- the second and third steps are repeated to obtain homogeneous and at least partial sterilisation or homogeneous and complete pasteurisation of said substrate.

2. The method according to claim 1, wherein the second step is effected by transporting the substrate in the first direction within the tubular section by means of transport device situated substantially inside said tubular section.

3. The method according to claim 2, wherein the substrate is discharged from the vessel by reversing the direction of the transport device.

4. The method according to any of claims 1 to 3, wherein during the transport in the second direction the substrate is subjected to tumbling.

5. A method according to any of claims 1 to 4, wherein the homogeneously completely pasteurised or at least partly sterilised substrate is subjected to a cooling step in the vessel.

6. The method according to any previous claim further comprising the step of introducing a fluid for moistening said substrate.

7. The method according to claim 6, wherein the quantity of moistening fluid introduced in the first step is such that the concentration of dry material in the sealed rotary machine is from 20% by weight to 60% by weight.

8. The method according to any of the previous claims wherein the hollow vessel is sealed during sterilisation or pasteurisation.

9. The method according to any previous claim, wherein the substrate is an organic material.

10. The method according to claim 9, wherein the substrate is a growth or nutrient medium.

11. The method according to claim 10, wherein the growth medium is seeded inside the vessel, after it has cooled, before the step of discharging it from the vessel.

12. Apparatus for at least partly sterilising or pasteurising a substrate, comprising:
- a hollow vessel provided with means for its rotation about a rotation axis, and with means for at least partly sterilising or completely pasteurising the substrate;
- an opening for loading the substrate into the vessel;
- a first transport device for transporting the substrate in a first direction and extending throughout a substantial portion of the interior of the hollow vessel,
- a tubular partition surrounding the first transport device and having a wall forming a separator between a zone comprised inside the tubular partition and a zone comprised between said tubular partition and the inner wall of the hollow vessel,
- a means for discharging the at least partly sterilised or completely pasteurised substrate from the tubular partition into a hollow space of the hollow vessel, and
- a second transport device for directing the substrate discharged from the tubular partition to follow a path in a second direction opposite to the first direction.

13. The apparatus according to claim 12, wherein the tubular partition extends over at least a majority portion of the length of the first transport device.

14. The apparatus according to claim 12 or 13, further comprising means for introducing a moistening fluid into the vessel.

15. The apparatus according to any of claims 12 to 14, further comprising means for discharging the substrate from the vessel.

16. The apparatus according to claim 15, wherein the discharging means comprises a reverse drive for the first transport device.

17. The apparatus according to any of the claims 12 to 16, wherein the vessel is provided with a means for sealing during processing.

18. The apparatus according to any of claims 12 to 17, wherein the tubular partition and the hollow vessel are elongate, cylindrical or quasi-cylindrical, parallelepiped or quasi-parallelepiped.

19. The apparatus according to any of the claims 12 to 18, wherein the tubular partition comprises a means for preventing clogging of the substrate within the tubular partition.

20. The apparatus according to any of the claims 12 to 19 wherein the rotation axis of the vessel is substantially horizontal or with a tilt of less than 25° and preferably less than 10°.

21. The apparatus according to any of the claims 12 to 20 wherein, the means for sterilising or pasteurising the substrate comprises a fluid circulation jacket around the tubular partition and/or a fluid circulation envelope on a wall of the vessel.

22. The apparatus according to any of claims 12 to 21, wherein the first transport device is a screw, worm spiral conveyor.

23. The apparatus according to any of the claims 12 to 22 further comprising a second transporting device for transporting the substrate in the second direction.

24. The apparatus according to claim 23, wherein the second transporting device is adapted to allow tumbling of the substrate in the vessel.

25. The apparatus according to any of the claims 12 to 24, wherein the first transport device is partly surrounded by a trough which is coaxial with the tubular partition.

## Patentansprüche

1. Diskontinuierliches oder chärgenweises Verfahren um, ein Substrat zumindest teilweise zu sterilisieren oder homogen und vollständig zu pasteurisieren, wobei das Verfahren folgendes umfasst:
- in einem ersten Schritt wird ein Substrat in ein Hohlgefäß eingefüllt, wobei das Hohlgefäß mit Mitteln ausgestattet ist, um das Substrat zumindest teilweise zu sterilisieren oder zu pasteurisieren;
- in einem zweiten Schritt wird das Hohlgefäß rotiert und Wärmeenergie wird dem Substrat zugeführt, wobei das Substrat während der Rotation des Hohlgefäßes zu einem Ende des Hohlgefäßes in einer ersten Richtung durch einen rohrförmigen Abschnitt transportiert wird, der sich im Hohlgefäß befindet, wobei der rohrförmige Abschnitt ein erstes und ein zweites Ende aufweist;
- in einem dritten Schritt wird das Substrat aus dem zweiten Ende des rohrförmigen Abschnittes im rotierenden Hohlgefäß ausgetragen und wird in eine zweite Richtung transportiert, die der ersten Richtung im Hohlgefäß entgegengesetzt ist, bis es das erste Ende des rohrförmigen Abschnitts erreicht, wobei das in die erste Richtung transportierte Substrat von dem in die zweite Richtung transportierten Substrat durch eine Wand des rohrförmigen Abschnitts getrennt ist; und
- die zweiten und dritten Schritte werden wiederholt, um eine homogene und zumindest teilweise Sterilisation oder homogene und vollständige Pasteurisierung des Substrates zu erreichen.

2. Verfahren nach Anspruch 1, wobei der zweite Schritt durch Transportieren des Substrates in der ersten Richtung im rohrförmigen Abschnitt mittels einer Transportvorrichtung bewirkt wird, die sich im wesentlichen innerhalb des rohrförmigen Abschnitts befindet.

3. Verfahren nach Anspruch 2, wobei das Substrat aus dem Gefäß ausgetragen wird, indem die Richtung der Transportvorrichtung umgekehrt wird.

4. Verfahren nach einem der Ansprüche 1 - 3, wobei während des Transports in der zweiten Richtung das Substrat einem Stürzen unterworfen wird.

5. Verfahren nach einem der Ansprüche 1 - 4, wobei das homogen, vollständig pasteurisierte oder zumindest teilweise sterilisierte Substrat einem Abkühlschritt im Gefäß unterworfen wird.

6. Verfahren nach einem der vorhergehenden Ansprüche, das weiterhin den Schritt umfasst, eine Flüssigkeit zum Befeuchten des Substrates einzubringen.

7. Verfahren nach Anspruch 6, wobei die Menge der Befeuchtungsflüssigkeit, die im ersten Schritt eingebracht wird derart ist, dass die Konzentration an Trockenmaterial in der verschlossenen Rotationsmaschine von 20 Gew.-% bis 60 Gew.-% beträgt.

8. Verfahren nach einem der vorhergehenden Ansprüche, wobei das Hohlgefäß während der Sterilisation oder Pasteurisation verschlossen ist.

9. Verfahren nach einem der vorhergehenden Ansprüche, wobei das Substrat ein organisches Material ist.

10. Verfahren nach Anspruch 9, wobei das Substrat ein Wachstums- oder Nährmedium ist.

11. Verfahren nach Anspruch 10, wobei das Wachstumsmedium im Gefäß beimpft wird, nachdem es abgekühlt ist, vor dem Schritt, es aus dem Gefäß auszutragen.

12. Vorrichtung zum zumindest teilweise Sterilisieren oder Pasteurisieren des Substrates, welche aufweist:
- ein Hohlgefäß, das mit Mitteln zu seiner Drehung um eine Rotationsachse und mit Mitteln zum zumindest teilweise Sterilisieren oder vollständigen Pasteurisieren des Substrats ausgestattet ist;
- eine Öffnung zum Einfüllen des Substrates in das Gefäß;
- eine erste Transportvorrichtung zum Transportieren des Substrates in eine erste Richtung, die sich durch einen wesentlichen Anteil des Innenraums des Hohlgefäßes erstreckt,
- einen rohrförmigen Abschnitt, der die erste Transportvorrichtung umgibt und eine Wand aufweist, die einen Separator zwischen einer Zone, die sich innerhalb des rohrförmigen Anteils befindet, und einer Zone bildet, die sich zwischen dem rohrförmigen Anteil und der Innenwand des Hohlgefäßes befindet,
- ein Mittel zum Austragen des zumindest teilweise sterilisierten oder vollständig pasteurisierten Substrates aus dem rohrförmigen Anteil in einen Hohlraum des Hohlgefäßes, und
- eine zweite Transportvorrichtung, um das Substrat, das aus dem rohrförmigen Anteil ausgetragen wird, so zu führen, dass es einem Weg in einer zweiten Richtung folgt, die der ersten Richtung entgegengesetzt ist.

13. Vorrichtung nach Anspruch 12, wobei der rohrförmige Anteil sich über zumindest einen Hauptanteil der Länge der ersten Transportvorrichtung erstreckt.

14. Vorrichtung nach Anspruch 12 oder 13, die weiterhin ein Mittel zum Einbringen einer Befeuchtungsflüssigkeit in das Gefäß aufweist.

15. Vorrichtung nach einem der Ansprüche 12 bis 14, die weiterhin ein Mittel zum Austragen des Substrates aus dem Gefäß aufweist.

16. Vorrichtung nach Anspruch 15, wobei die Austragsmittel einen Umkehrantrieb für die erste Transportvorrichtung aufweist.

17. Vorrichtung nach einem der Ansprüche 12 bis 16, wobei das Gefäß mit einem Mittel zum Verschließen während der Verarbeitung ausgestattet ist.

18. Vorrichtung nach einem der Ansprüche 12 bis 17, wobei der rohrförmige Abschnitt und das Hohlgefäß länglich, zylindrisch oder quasi-zylindrisch, parallelepiped oder quasi-parallelepiped sind.

19. Vorrichtung nach einem der Ansprüche 12 bis 18, wobei der rohrförmige Abschnitt ein Mittel zum Vorbeugen einer Verstopfung des Substrates im rohrförmigen Abschnitt aufweist.

20. Vorrichtung nach einem der Ansprüche 12 - 19, wobei die Rotationsachse des Gefäßes im wesentlichen horizontal ist oder eine Neigung von weniger als 25° und vorzugsweise weniger als 10° aufweist.

21. Vorrichtung nach einem der Ansprüche 12 - 20, wobei das Mittel zum Sterilisieren und Pasteurisieren des Substrates einen Flüssigkeitszirkulationsmantel um den rohrförmigen Anteil und/oder eine Flüssigkeitszirkulationsumhüllung auf einer Wand des Gefäßes aufweist

22. Vorrichtung nach einem der Ansprüche 12 - 21, wobei die erste Transportvorrichtung ein Schnecken-, Wurm-Spiralförderer ist.

23. Vorrichtung nach einem der Ansprüche 12 - 22, die weiterhin eine zweite Transportvorrichtung zum Transportieren des Substrates in die zweite Richtung aufweist.

24. Vorrichtung nach Anspruch 23, wobei die zweite Transportvorrichtung ein Stürzen des Substrates im Gefäß erlaubt.

25. Vorrichtung nach einem der Ansprüche 12 - 24, wobei die erste Transportvorrichtung teilweise von einer Wanne umgeben ist, die mit dem rohrförmigen Abschnitt coaxial ist.

## Revendications

1. Procédé discontinu - ou par lot - pour stériliser au moins partiellement ou pasteuriser de manière homogène et complètement un substrat, procédé dans lequel :
- dans une première étape, ledit substrat est chargé dans un récipient creux, le récipient creux étant muni d'un moyen pour stériliser au moins en partie ou pasteuriser le substrat ;
- dans une deuxième étape, le récipient creux est mis en rotation et de l'énergie calorifique est fournie au substrat, pendant la rotation du récipient creux, le substrat étant transporté vers une extrémité du récipient creux dans un premier sens à travers une section tubulaire située à l'intérieur du récipient creux, la section tubulaire ayant une première et une seconde extrémité ;
- dans une troisième étape, le substrat est déchargé de la seconde extrémité de la section tubulaire dans le récipient creux rotatif et est transporté dans un second sens opposé à celui du premier sens dans le récipient creux jusqu'à ce qu'il atteigne la première extrémité de la section tubulaire, le substrat transporté dans le premier sens étant séparé du substrat transporté dans le second sens par une paroi de la section tubulaire ; et
- les deuxième et troisième étapes sont répétées pour obtenir une stérilisation homogène et au moins partielle ou une pasteurisation homogène et complète dudit substrat.

2. Procédé selon la revendication 1, dans lequel la deuxième étape est effectuée en transportant le substrat dans le premier sens à l'intérieur de la section tubulaire au moyen d'un dispositif de transport placé sensiblement à l'intérieur de ladite section tubulaire.

3. Procédé selon la revendication 2, dans lequel le substrat est déchargé du récipient en inversant le sens du dispositif de transport.

4. Procédé selon l'une quelconque des revendications 1 à 3, dans lequel, pendant le transport dans le second sens, le substrat est soumis à un tonnelage.

5. Procédé selon l'une quelconque des revendications 1 à 4, dans lequel le substrat pasteurisé complètement de façon homogène ou au moins stérilisé en partie est soumis à une étape de refroidissement dans le récipient.

6. Procédé selon l'une quelconque des revendications précédentes, comprenant de plus l'étape consistant à introduire un fluide pour humidifier ledit substrat.

7. Procédé selon la revendication 6, dans lequel la quantité de fluide d'humidification introduit dans la première étape est telle que la concentration de matière sèche dans la machine rotative scellée est comprise entre 20 % en poids à 60 % en poids.

8. Procédé selon l'une quelconque des revendications précédentes, dans lequel le récipient creux est scellé pendant la stérilisation ou la pasteurisation.

9. Procédé selon l'une quelconque des revendications précédentes, dans lequel le substrat est une matière organique.

10. Procédé selon la revendication 9, dans lequel le substrat est un milieu de culture ou nutritif.

11. Procédé selon la revendication 10, dans lequel le milieu de culture est semé à l'intérieur du récipient, après qu'il a refroidi, avant l'étape de déchargement de ce dernier hors du récipient.

12. Appareil pour au moins stériliser en partie ou pasteuriser un substrat, comprenant :
- un récipient creux muni d'un moyen pour sa rotation autour d'un axe de rotation, et d'un moyen pour stériliser au moins en partie ou pour pasteuriser complètement le substrat ;
- une ouverture pour charger le substrat dans le récipient ;
- un premier dispositif de transport pour transporter le substrat dans un premier sens et s'étendre tout au long d'une partie importante de l'intérieur du récipient creux ;
- une séparation tubulaire entourant le premier dispositif de transport et ayant une paroi formant un séparateur entre une zone comprise à l'intérieur de la séparation tubulaire et une zone comprise entre ladite séparation tubulaire et la paroi intérieure du récipient creux ;
- un moyen pour décharger le substrat stérilisé au moins en partie ou complètement pasteurisé en dehors de la séparation tubulaire dans un espace creux du récipient creux ; et
- un second dispositif de transport pour diriger le substrat déchargé en dehors de la séparation tubulaire pour suivre un chemin dans un second sens opposé au premier sens.

13. Appareil selon la revendication 12, dans lequel la séparation tubulaire s'étend sur au moins une partie principale de la longueur du premier dispositif de transport.

14. Appareil selon la revendication 12 ou 13, comprenant de plus un moyen pour introduire un fluide d'humidification dans le récipient.

15. Appareil selon l'une quelconque des revendications 12 à 14, comprenant de plus un moyen pour décharger le substrat hors du récipient.

16. Appareil selon la revendication 15, dans lequel le moyen de déchargement comprend une commande d'inversion pour le premier dispositif de transport.

17. Appareil selon l'une quelconque des revendications 12 à 16, dans lequel le récipient est muni d'un moyen de scellement pendant le traitement.

18. Appareil selon l'une quelconque des revendications 12 à 17, dans lequel la séparation tubulaire et le récipient creux sont allongés, cylindriques ou presque cylindriques, parallélépipédiques ou presque parallélépipédiques.

19. Appareil selon l'une quelconque des revendications 12 à 18, dans lequel la séparation tubulaire comprend un moyen pour empêcher l'obstruction du substrat à l'intérieur de la séparation tubulaire.

20. Appareil selon l'une quelconque des revendications 12 à 19, dans lequel l'axe dé rotation du récipient est sensiblement horizontal ou avec une inclinaison inférieure à 25° et de préférence inférieure à 10°.

21. Appareil selon l'une quelconque des revendications 12 à 20, dans lequel, le moyen pour stériliser ou pasteuriser le substrat comprend une gaine de circulation de fluide autour de la séparation tubulaire et/ou une enveloppe de circulation de fluide sur une paroi du récipient.

22. Appareil selon l'une quelconque des revendications 12 à 21, dans lequel le premier dispositif de transport est un transporteur à vis hélicoïdale sans fin.

23. Appareil selon l'une quelconque des revendications 12 à 22, comprenant de plus un second dispositif de transport pour transporter le substrat dans le second sens.

24. Appareil selon la revendication 23, dans lequel le second dispositif de transport est conçu pour permettre le tonnelage du substrat dans le récipient.

25. Appareil selon l'une quelconque des revendications 12 à 24, dans lequel le premier dispositif de transport est entouré en partie par une cuve qui est coaxiale avec la séparation tubulaire.
